# EUROPÄISCHE PATENTSCHRIFT

(11) **EP 2 566 524 B1**
(45) Veröffentlichungstag und Bekanntmachung des Hinweises auf die Patenterteilung: **17.02.2021**
(21) Anmeldenummer: 11724984.7
(22) Anmeldetag: 09.05.2011
(51) Int. Cl.: A61L 2/14, B67C 7/00, B29C 49/00

(54) **PLASMAGENERIERTES GAS-STERILISATIONSVERFAHREN**
PLASMA-GENERATED GAS STERILIZATION METHOD
PROCÉDÉ DE STÉRILISATION PAR UN GAZ GÉNÉRÉ PAR UN PLASMA

(30) Priorität: 07.05.2010 DE 102010019863
(43) Veröffentlichungstag der Anmeldung: 13.03.2013
(73) Patentinhaber: Leibniz-Institut für Plasmaforschung und Technologie e.V., 17489 Greifswald (DE)
(72) Erfinder: KROHMANN, Udo, 17033 Neubrandenburg (DE); EHLBECK, Joerg, 17498 Hinrichshagen (DE); NEUMANN, Torsten, 17033 Neubrandenburg (DE); SCHNABEL, Uta, 17491 Greifswald (DE); ANDRASCH, Mathias, 18439 Stralsund (DE); LEHMANN, Wolfram, 17498 Neuenkirchen (DE); WELTMANN, Klaus-Dieter, 18609 Ostseebad Binz (DE)
(74) Vertreter: Schulz Junghans Patentanwälte PartGmbB
(86) Internationale Anmeldenummer: PCT/EP2011/057402
(87) Internationale Veröffentlichungsnummer: WO 2011/138463

(56) Entgegenhaltungen:
- WO-A2-96/21473
- WO-A2-2005/023319
- DE-A1- 19 827 442
- GB-A- 2 302 654
- SINGH M K ET AL: "Study of inactivation factors in low temperature surface-wave plasma sterilization", DENKI GAKKAI RONBUNSHI. A, KISO, ZAIRYO, KYOTSU BUMONSHI -TRANSACTIONS OF THE INSTITUTE OF ELECTRICAL ENGINEERS OF JAPAN. A, DENKI GAKKAI, TOKYO, JP, Bd. 129, Nr. 1, 1. Januar 2009 (2009-01-01), Seiten 30-34, XP009150829, ISSN: 0385-4205, DOI: 10.1541/IEEJFMS.129.30

## Beschreibung

Die Erfindung betrifft ein Verfahren und eine Vorrichtung zur schnellen Dekontamination und Sterilisation von vorzugsweise thermolabilen Gütern unter Benutzung eines vorzugsweise aus Luft als Prozessgas generierten Plasmagases und dessen anschließender Befeuchtung mit Wasser.

Bekannte Sterilisationsverfahren sind die Autoklavierung, das heißt die Anwendung feuchter Hitze, die Bestrahlung mit ionisierenden Strahlen, die Gassterilisation mit Ethylenoxid (ETO), die Plasmasterilisation und die Wasserstoffperoxidsterilisation.

Das Autoklavieren erzielt eine sehr gute Sterilisationswirkung, kann jedoch für thermolabile Güter nicht eingesetzt werden.

Ethylenoxid (ETO) ist ein hochtoxisches Gas, das zur Sterilisation thermolabiler Materialien eingesetzt wird, dessen Nachteil allerdings auf den relativ langen Ausgasungszeiten der zu sterilisierenden Güter beruht, wobei die Ausgasungszeiten um ein Vielfaches höher liegen können als die eigentliche Behandlungszeit. Aufgrund der Toxizität ist der Umgang mit ETO kritisch.

Nachteile der Wasserstoffperoxidsterilisation sind in erster Linie darin zu sehen, dass es sich bei Wasserstoffperoxid bei Raumtemperatur um eine Flüssigkeit handelt. Die bekannten Verfahren basieren auf einer Verdampfung des Wasserstoffperoxides, wobei allerdings eine Kondensation im Bereich der zu sterilisierenden Gegenstände vermieden werden muss, um die Dampfphase vollständig abziehen zu können. Die Entfernung des Wasserstoffperoxides gelingt nur schlecht, wenn sich größere Flüssigkeitsmengen angesammelt haben. Die Verdampfung ist in diesem Fall ungleichmäßig und dauert lange.

Die Behandlung mit ionisierender Strahlung setzt einen großen apparativen und sicherheitstechnischen Aufwand voraus. Zum anderen kann ionisierende Strahlung das Material der sterilisierenden Gegenstände gegebenenfalls beschädigen. Die bekannten Plasmasterilisationsverfahren weisen diese Nachteile nicht oder nur in geringem Maße auf. Die sterilisierende Wirkung eines Plasmas beruht unter anderem auf einer Zerstörung der Keime und Biomoleküle durch die Kombination von UV- und VUV-Strahlung, welches die DNA zerstört. Nachteilig ist, dass eine Sterilisation von temperaturempfindlichen Gütern in der Regel erst bei zunehmendem Unterdruck möglich ist.

Es hat sich jedoch gezeigt, dass bisher bekannte Plasmaverfahren nur bedingt zur Behandlung bzw. Dekontamination und Sterilisation komplexer Strukturen geeignet sind, da z.B. das Eindringvermögen der Plasmen in enge Spalten und Lumen, selbst unter Hochvakuum, nicht sehr gut ist.

Problematisch ist auch die Behandlung mit sterilisierenden Gasen und Stoffen (z.B. Ethylenoxid und Wasserstoffperoxid), die in höheren Konzentrationen zum Teil hochexplosiv sind. Auch erfordern diese Verfahren längere Nachbehandlungsphasen zur Beseitigung von Resten der hochtoxischen Stoffe. Somit sind die Lagerung und der Umgang mit diesen Stoffen für den Anwender kompliziert und teuer.

In US 2008/0317626 A1 ist ein Verfahren und eine Vorrichtung beschrieben, in dem eine Sterilgas generierende Verbindung (C-basierte diazeniumdiolate Verbindung und eine pulverförmige Säure) genutzt wird, welche als Sterilgas vorzugsweise NO oder eine Mischung aus NO und NO₂ generiert.

In US 2010/0166603 A1 ist ein Sterilisationsverfahren für Pulver unter Verwendung von NO₂ und Feuchtigkeit als sterilisierendes Gas beschrieben. Als Quelle für gasförmiges NO₂ wird flüssiges NO₂ verwendet.

Nachteilig für diese Verfahren ist, dass auch hier chemische Substanzen gelagert und für die Bildung der erforderlichen Wirkgase eingesetzt werden. Erforderliche Wirkzeiten liegen bei einer Stunde und darüber.

In WO 2010/022871 ist ein nichtthermisches Plasmasterilisationsverfahren beschrieben, welches u.a. Luft als Trägergas und die Verwendung verschiedener Zusatzstoffe beschreibt. Als wirksame Spezies werden z.B. NOₓ und H₂O₂ genannt. Die Verwendung eines nichtthermischen Plasmas ermöglicht zwar grundsätzlich die Generierung von antimikrobiell wirkenden Spezies und damit auch eine gewisse Abtötungskinetik von einigen log-Stufen, eine sterilisierende Wirkung für die wichtigsten Referenzkeime und Sporen kann jedoch nicht durch die geringe Produktion der keimtötenden Spezies erreicht werden. Eine sichere und reproduzierbare Sterilisation, insbesondere auch bei größeren Durchsätzen von zu sterilisierenden Gütern, ist mit diesem Verfahren nicht umsetzbar.

Bekannt ist die sterlisierende Wirkung von plasmageneriertem Gas, wie auch in der Patentanmeldung US 2010/0254853 A1 beschrieben. Die Wirkung von plasmageneriertem Gas beruht im Wesentlichen auf der Generierung von NOₓ und freien Radikale mit den damit verbundenen langen Wirkzeiten von einer Stunde und darüber. GB2302654A offenbart eine Atmosphärendruckplasmaerzeugung ausserhalb der Sterilisationskammer mit nachgeschalter Ozonentfernung noch vor Einleitung in die Sterilisationskammer.

Der vorliegenden Erfindung liegt die Aufgabe zugrunde, die Nachteile der im Stand der Technik beschriebenen Lösungen zu beseitigen.

Die Aufgabe wurde gemäß den Merkmalen der Patentansprüche gelöst. Erfindungsgemäß wurde sowohl ein schnelles und kostengünstiges Verfahren, als auch eine Vorrichtung zur Sterilisation von Gütern bereitgestellt, mit denen:
a) die zur Sterilisation wirksamen Spezies in ausreichender Menge vorzugsweise unter Verwendung von Luft erzeugt werden, sowie
b) eine effiziente und schonende Behandlung auch von strukturierten Gütern mit engen Spalten und Kapillaren ermöglicht wird und
c) eine Sterilisation von Gütern und Stoffen in wenigen Sekunden erfolgen kann.

Überraschenderweise hat sich herausgestellt, dass insbesondere dieses Verfahren auch für eine kostengünstige und effiziente Sterilisation von preiswerten medizinischen Massenprodukten und Verpackungsmitteln geeignet ist.

Das erfindungsgemäße Verfahren besteht darin, dass der Ionisationsprozess des Prozessgases Luft, von dem eigentlichen Sterilisationsprozess räumlich getrennt erfolgt, indem für die Sterilisation wirksame Spezies sowohl in der Ionisationsphase als auch in der sich anschließenden Rekombinations- und Abkühlphase erzeugt werden und nur das sich hieraus bildende plasmaaktivierte Gasgemisch für die Sterilisation genutzt wird, eine direkte Plasmabehandlung somit nicht erfolgt.

Das Verfahren zur Dekontamination und Sterilisation von Gegenständen und Gütern ist durch Anspruch 1 definiert und umfasst unter anderem die folgenden Schritte:
a) Erzeugung eines Plasmas vorzugsweise aus Luft als Prozessgas, welches reaktive Stickstoff- und Sauerstoffspezies bildet
b) Oxidation von NO zu NO2, bei Temperaturen unterhalb von 400 °C, so dass sich ein plasmaaktiviertes Gasgemisch mit mindestens 0,3% NO₂-Anteil bildet
c) Inkontaktbringen dieses plasmaaktivierten Gasgemischs mit Wasser in einem oder mehreren seiner Aggregatzustände
d) Inkontaktbringen des unter Punkt c) erzeugten Gasgemischs mit den zu dekontaminierenden bzw. zu sterilisierenden Gegenständen und Gütern

Hierzu wird vorzugsweise trockene Luft als Prozessgas durch eine, vorzugsweise ein Volumenplasma bildende Plasmaquelle (z.B. Mikrowellenplasma) geführt und teilweise ionisiert, wobei üblicherweise unter Atmosphärendruck bei der Erzeugung ein heißes Luftplasma mit einer Gastemperatur von mindestens 1200 °C erzeugt wird. Nach Austreten des Plasmas aus dem Anregungsbereich der Plasmaquelle wird durch eine spezifizierte Abkühlung des Luftplasmas die Bildung eines aktivierten Plasmagases veranlasst, welches gegenüber dem Ausgangsgas Luft, nach einer gewissen Reaktionszeit einen Anteil von mindestens 0,3 % NO₂ enthält.

Es entspricht dem heutigen Stand der Technik durch numerische Simulation den Abkühlprozess in Abhängigkeit der erforderlichen Ausgangsspezies zu spezifizieren. Durch geeignete und bekannte Kühlmechanismen wird das Plasmagas je nach Erfordernis, mindestens jedoch unter 400 °C abgekühlt und ein plasmaaktiviertes Gasgemisch mit. NO₂-Konzentrationen von typischerweise zwischen 0,3 und 5% gebildet. Durch eine Befeuchtung des plasmaaktivierten Gasgemischs mit Wasser in einem oder mehreren seiner Aggregatzustände in einem Temperaturbereich von -40 °C bis 400 °C wird eine teilweise Reaktion von Wasser mit Bestandteilen des Gasgemischs ermöglicht. Die Kontaktzeit des plasmaaktivierten Gasgemischs mit Wasser kann von Millisekunden bis zu Stunden betragen. Dieses sich durch die Reaktion mit Wasser gebildete Gasgemisch (sterilisierendes Gasgemisch) als auch die aus der Reaktion des plasmaaktivierten Gasgemischs mit dem Wasser sich bildende Lösung (sterilisierende Lösung) können als sterilisierende Agens verwendet werden. Die Befeuchtung des plasmaaktivierten Gasgemischs mit Wasser kann bis zur Sättigung erfolgen.

Die zu sterilisierenden Güter werden nun dem mit Wasser befeuchteten Gasgemisch in einer vorgegeben Zeit zur Wirkung ausgesetzt. Das mit Wasser befeuchtete sterilisierende Gasgemisch wird hierzu entweder über die zu sterilisierenden Güter geleitet oder die Güter befinden sich in einer Prozesskammer, in die das mit Wasser befeuchtete sterilisierende Gasgemisch eingeleitet wird. Durch Veranlassung einer Mikrokondensation von gasförmigen Verbindungen auf der kühleren Substratoberfläche kann eine wesentliche Verkürzung der Behandlungszeit und Erhöhung der Wirksamkeit erreicht werden. Durch Verwendung einer Düse am Ausgang der Plasmaquelle kann die schlagartige Expansion des Plasmagases nach Austritt aus der Düse zur Abkühlung und durch Unterschreitung der Taupunkttemperatur zur Mikrokondensation genutzt werden. Das zu sterilisierende Gut kann auch teilweise oder ganz zur Abkühlung des Plasmagases oder zur Mikrokondensation genutzt werden.

In Abhängigkeit von dem gewünschten Grad der Dekontamination ist eine Wirkzeit (Verweilzeit der zu sterilisierenden Güter im sterilisierenden Gasgemisch) von ca. 2 Sekunden bis zu 60 Min. je nach Applikation einzustellen.

Die Einstellung der gewünschten Befeuchtung mit Wasser kann durch bekannte Vorrichtungen wie z.B. Verdampfer, Bubbler, Ultraschallnebler, Eindüsung von Wasser oder Wasserdampf sowie mittels Eisdosierer oder Vereiser erfolgen. Die Reaktion des plasmaaktivierten Gasgemischs mit Wasser kann auch in einem Puffergefäß erfolgen, welches teilweise mit Wasser gefüllt ist, auch kann das plasmaaktivierte Gasgemisch durch das Wasser durchgeleitet werden.

Nach Beendigung der vorgegebenen Wirkzeit wird die Prozesskammer mit Sterilluft so lange gespült bis die zulässigen MAK-Werte unterschritten sind. Eine Entsorgung des sterilisierenden Gases kann durch bekannte geeignete Maßnahmen, wie z.B. durch Einsatz eines Gaswäschers oder Absorbermaterials realisiert werden.

Zur Verbesserung der Wirkung in engen Lumen und Spalten kann die Prozesskammer vor Einleiten des sterilisierenden Gasgemisches evakuiert werden. Durch Einleiten des sterilisierenden Gasgemisches bis zum Erreichen des Atmosphärendrucks und darüber werden auch enge Lumen und Spalten sicher von dem bioziden Gasgemisch erreicht. Durch mehrfaches Anwenden einer Druckänderung während des Wirkprozesses des sterilisierenden Gases (Druckwechseltechnologie) kann der Sterilisationsprozess beschleunigt und die Funktionssicherheit insbesondere in Kavitäten erhöht werden. Durch eine höhere Anzahl von Druckzyklen kann die Druckdifferenz der Zyklen reduziert werden. Somit können auch druckempfindliche Güter behandelt werden.

Weiterhin kann das sterilisierende Gas durch die Prozesskammer geführt und anschließend wieder der Plasmaquelle zugeführt werden, so dass im Kreislaufprozess die Konzentration der bioziden Bestandteile des Plasmagases bis zur erforderlichen Konzentration kontinuierlich erhöht werden kann.

Zur Behandlung von Gütern, die ganz oder teilweise aus Hohlräumen bestehen, kann das sterilisierende Gas direkt in oder durch die Hohlräume geleitet werden.

Eine sterilisierende Gasmischung kann auch dadurch erzeugt werden, indem Luft durch die bereits vorher hergestellte sterilisierende Lösung (15), hergestellt aus der Reaktion von plasmaaktiviertem Gasgemisch mit Wasser, geleitet wird und das sich daraus bildende befeuchtete Gasgemisch zur Sterilisation genutzt wird.

Eine Vorrichtung zur Durchführung des Verfahrens ist durch Anspruch 9 definiert und besteht unter anderem aus einer Zuführeinrichtung für Luft, einer Plasmaquelle zur Erzeugung eines Plasmas, einer Vorrichtung zur Oxidierung für das aus der Plasmaquelle austretende Luftplasma, einer Befeuchtungsvorrichtung mit Wasser und einer Prozesskammer für die Aufnahme der zu dekontaminierenden oder sterilisierenden Güter. Durch zusätzliche Anordnung einer Vakuumpumpe bzw. einer Umluftfördereinheit oder eines Kompressors für das sterilisierende Gas kann die Vorrichtung zweckmäßig ergänzt werden.

Unter plasmaaktiviertes Gasgemisch ist im Sinne der Erfindung das sich nach einer Ionisation und anschließender Rekombination sowie Abkühlung bildende Gasgemisch zu verstehen, welches gegenüber dem Ausgangsgas neue Molekülverbindungen und Radikale beinhaltet.

Die Vorteile des erfindungsgemäßen Verfahrens und Vorrichtung gegenüber dem Stand der Technik liegen insbesondere darin begründet, dass eine sehr schnelle Inaktivierung der Mikroorganismen bis hin zur Sterilisation in wenigen Sekunden ermöglicht wird sowie eine direkte Plasmakontaktierung der zu behandelnden Stoffe und Gegenstände nicht erfolgt resp. erfolgen muss und für die Erzeugung der für die Dekontamination wichtigen Spezies vorzugsweise nur Raumluft resp. Umgebungsluft als Arbeitsgas und Wasser verwendet wird. Somit können selbst temperaturlabile Stoffe problemlos behandelt werden. Durch die zusätzliche kombinierte Wirkung von gasförmigen und flüssigen (Kondensat) Spezies werden extrem kurze und hochwirksame Behandlungszeiten der zu dekontaminierenden oder zu sterilisierenden Güter ermöglicht. Eine Materialschädigung kann somit weitestgehend vermieden werden. Durch die Trennung des Plasmaprozesses von dem Sterilisationsprozess ist eine direkte Wirkung des Plasmas auf die Materialeigenschaften der zu sterilisierenden Güter und somit auch eine Rückkopplung auf den Plasmaprozess ausgeschlossen, welches zu einer wesentlichen Erhöhung der Prozesssicherheit führt.

Eine extreme Abkühlung des sterilisierenden Gasgemisches auf ca. 40 °C und tiefer ermöglicht somit auch eine zeitlich länger andauernde nicht unter den beanspruchten Gegenstand fallende Humananwendung, z.B. zur Desinfektion von Wunden und eine Behandlung von Lebensmitteln.

Mit der Umsetzung dieses Verfahrens ist es erstmalig möglich, ein sehr heißes Luftplasma mit seinem hohen Wirkungsgrad bezüglich der Erzeugung von antimikrobiellen Spezies für die Sterilisation, auch von temperaturempfindlichen und strukturierten Gütern, zu nutzen, indem die Erzeugungs- von der Wirkphase durch den zwischengelagerten Kühlprozess voneinander getrennt wurde.

Dieses Verfahren arbeitet äußerst wirtschaftlich und effizient, da z.B. mit dem kostenlosen Arbeitsgas Luft sowie den geringen Mengen Wasser und nur einer Plasmaquelle mit einer Mikrowellenleistung von ca. 2kW bis zu ca. 4 m³ /h hochwirksames sterilisierendes Gasgemisch erzeugt werden kann. Dies ermöglicht beispielsweise eine Flaschendekontamination bei Zykluszeiten von ca. 2s pro Flasche. Da der Plasmaprozess und der Sterilisationsprozess voneinander getrennt sind, können mit einer Plasmaquelle nacheinander mehrere Prozesskammern mit dem sterilisierenden Gasgemisch gefüllt werden. Somit ergibt sich z.B. für Preforms die Möglichkeit, diese entweder in einem Hochratenprozess einzeln oder in einem Batchprozess in einer oder mehreren Kammern in großer Stückzahl zu desinfizieren.

Durch das hohe Diffusionsvermögen des sterilisierenden Gasgemisches, selbst durch gasdurchlässige Verpackungen (Tyvek), können strukturierte Güter mit engen Kapillaren und Spalten problemlos sterilisiert werden.

Das Verfahren kann durch Skalierung der eingespeisten Leistung und Anordnung mehrerer Plasmaquellen in einem großen Leistungsspektrum eingesetzt werden und eignet sich somit für kleine bis große Sterilisationsvolumen.

Dadurch, dass eine Gefahrgutlagerung von hochgiftigen Substanzen nicht erforderlich ist und prozessbedingt immer nur eine geringe Menge sterilisierendes Gasgemisch produziert wird, ist das Gefährdungspotenzial gegenüber Mensch und Umwelt auch im Falle einer Havarie der Anlage äußerst gering. Eine Anlagenüberwachung kann zum System gehören.

Die schnelle und hocheffiziente Wirksamkeit des Verfahrens konnte in mehreren mikrobiologischen Experimenten nachgewiesen werden.

Nachfolgend wird die Erfindung anhand von Ausführungsbeispielen erläutert.

### Ausführungsbeispiele

### Beispiel 1: Experiment zum Nachweis der antimikrobiellen Wirkung des plasmaaktivierten Gasgemisches

Nachfolgend ist ein Experiment beschrieben. Verschiedene Variationen sind möglich und für einige die mikrobizide Effektivität des Verfahrens in Tabelle 1 angegeben.

Dekontamination von verschiedenen vegetativen und sporolierten Mikroorganismen durch ein sterilisierendes Gasgemisch, erzeugt durch Verwendung von trockener oder angefeuchteter Luft (NO, NO₂) und Mikrowellenplasma.

Als Reaktionskammer, somit als Prüfkörper, wurden 250 ml Laborglasflaschen verwendet. Die Kontamination der Prüfkörper erfolgte im Sprühverfahren. Nach Trocknung der Prüfkörper erfolgte die Behandlung. Die Ausgangskonzentration lag jeweils bei 10⁸ KBE/ ml. Die Auswertung erfolgte mit standardisierten Methoden nach variierten Einwirkzeiten, 10 Sekunden bis 60 Minuten.

Die in der Tabelle 1 gezeigten Reduktionsfaktoren in log-Stufen (KBE/ ml) wurden nach den verschiedenen Nachwirkzeiten detektiert. Reduktionsfaktoren sind die Differenz zwischen dem Logarithmus der Kontrolle (log KBE) und dem Logarithmus der Anzahl der koloniebildenden Einheiten der Probe nach Einwirkung des plasmaaktivierten Gases. Die Nachweisgrenze lag bei 10⁻¹.

**Tab. 1: Reduktionsfaktoren vegetativer/ sporolierter Mikroorganismen nach verschiedenen Nachwirkzeiten des plasmaaktivierten Gasgemisches**

| | | | Reduktionsfaktoren (log[KBE/ ml]) nach Wirkzeit in Sekunden | | | | | | | | |
|---|---|---|---|---|---|---|---|---|---|---|---|
| Mikroorganismus | Gas | Tyvek | 10 | 20 | 30 | 60 | 300 | 600 | 900 | 1800 | 3600 |
| | | | | | | | | | | | |
| *E coli* | Luft | | | | | | >2 | >4 | >6 | >6 | >6 |
| | Luft | x | | | | | >1 | >1,5 | >3 | >6 | >6 |
| *S*. *aureus* | Luft | | | | | | | >4 | >5 | >6 | >6 |
| Konidiosporen von *A. brasiliensis* | Luft | x | | | | | >1,5 | >2 | >2 | >6 | >6 |
| Endosporen von *B*. *atrophaeus* | Luft | | | | | | >2 | >2 | >3 | >6 | >6 |
| | Luft/ H2O | | >6 | >6 | >6 | >6 | | | | | |
| | NO | | | | | | <1 | | | | |
| | NO/ H2O | | | | >1 | | | | | | |
| | NO2/ H2O | | | >6 | >6 | | | | | | |

Die Experimente zeigen eine Beschleunigung der mikrobiellen Inaktivierung um einen Faktor ≥ 10 unter Verwendung von Wasser. Zudem ist die Bedeutung von NO2 deutlich zu sehen. Für die zu erzielende Inaktivierung kann das antimikrobiell wirkende Gasgemisch mittels Plasma oder synthetisch hergestellt werden. Diffusionsbarrieren wie zum Beispiel Tyvek verlangsamen die Inaktivierung, aber inhibieren sie nicht. Eine Variation der Prüfkörper hat nur bei zunehmender Komplexität einen verzögernden Einfluss.

### Erläuterung der Vorrichtung anhand der Figuren

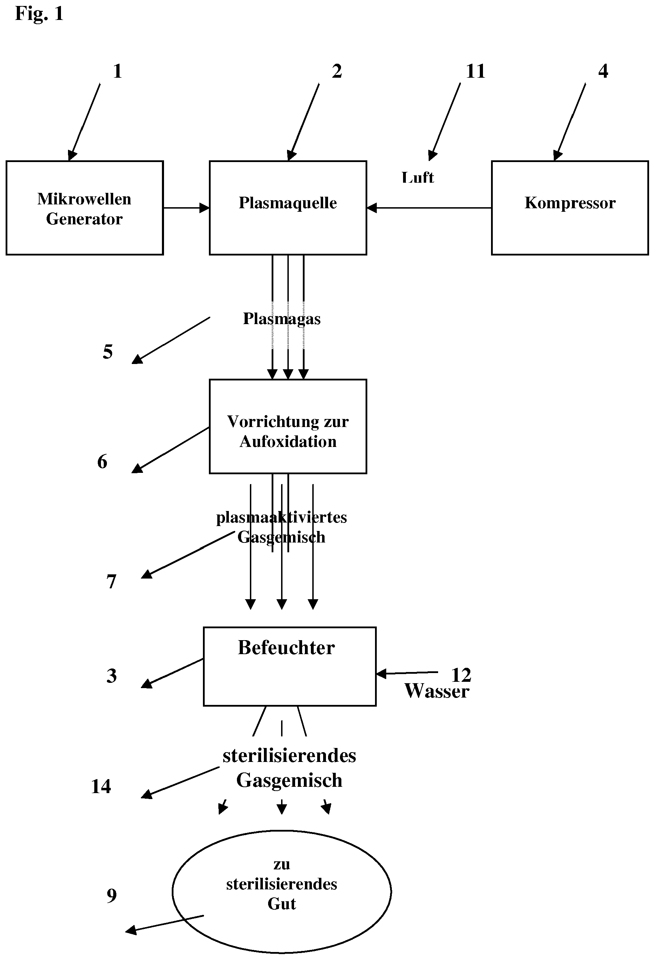

Fig. 1 zeigt eine Vorrichtung, mittels der aus dem Prozessgas Luft (11) ein biozides sterilisierendes Gasgemisch (14) erzeugt wird und das zu sterilisierende Gut (9) von dem sterilisierendes Gasgemisch (14) umströmt wird.

Die Vorrichtung besteht aus einem Mikrowellengenerator (1), der die Plasmaquelle (2) mit Mikrowellenenergie versorgt, der Plasmaquelle (2), die unter Atmosphärendruck ein voluminöses heißes Plasma (5) erzeugt, einem Kompressor (4), der die Luft (11) durch die Plasmaquelle (2) führt und einer Vorrichtung (Kühlvorrichtung) zur Oxidation von NO (6), mittels der das aus der Plasmaquelle (2) austretende heiße Plasmagas (5) soweit gekühlt wird, dass eine Oxidation des NO zu NO₂ erfolgt und ein plasmaaktiviertes Gasgemisch (7) entsteht, welches im Befeuchter (3) mit Wasser (12) befeuchtet wird, wodurch das sterilisierende Gasgemisch (14) entsteht, welches dem zu sterilisierenden Gut (9) zur Wirkung ausgesetzt wird, indem es von dem sterilisierenden Gasgemisch (14) umströmt wird.

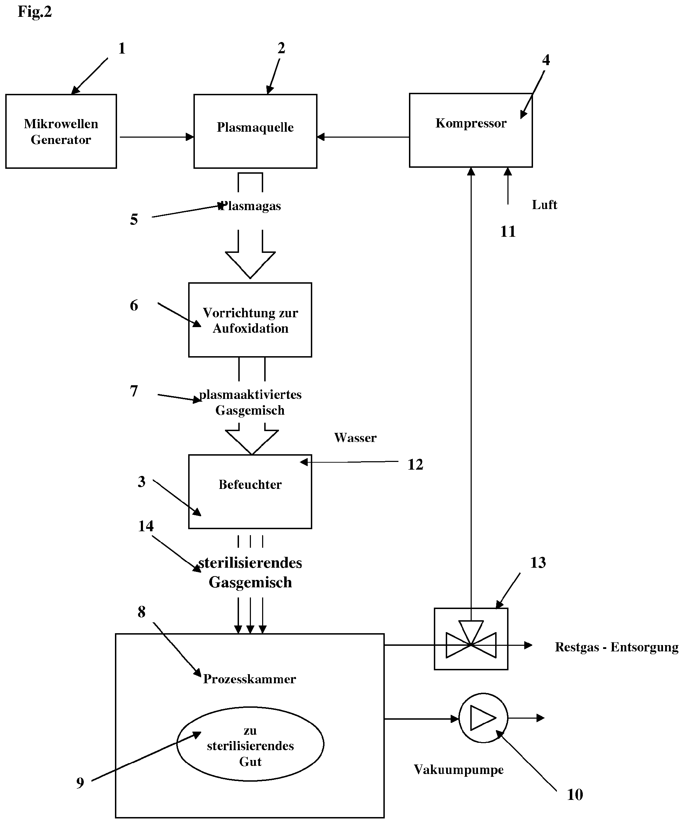

Fig. 2 zeigt eine Vorrichtung, mittels der aus dem Prozessgas Luft (11) ein biozides sterilisierendes Gasgemisch (14) erzeugt wird und das zu sterilisierende Gut (9) in einer Prozesskammer (8) dem sterilisierenden Gasgemisch (14) zur Wirkung ausgesetzt wird.

Die Vorrichtung besteht aus einem Mikrowellengenerator (1), einer Plasmaquelle (2), einem Kompressor (4), einem Befeuchter (3), einer Vorrichtung zur Oxidation von NO (6), einer Prozesskammer (8), einer Vakuumpumpe (10) sowie einer Stell- und Regeleinheit (13).

Der Mikrowellengenerator (1) versorgt die Plasmaquelle (2) mit Mikrowellenenergie, so dass aus der zugeführten Luft (11) vorzugsweise unter Atmosphärendruck ein heißes Plasma (5) generiert wird.

Mittels Vorrichtung zur Oxidation von NO (6) wird das aus der Plasmaquelle (2) austretende Plasmagas (5) in einer vorbestimmten Zeit soweit gekühlt, dass ein plasmaaktiviertes Gasgemisch (7) mit einem NO₂-Anteil von mindestens 0,5% gebildet wird. Dieses plasmaaktivierte Gasgemisch (7) wird in einem Befeuchter (3) mit Wasser (12) befeuchtet, so dass sich ein sterilisierendes Gasgemisch (14) bildet, welches in eine Prozesskammer (8) geleitet wird, in der sich das zu sterilisierende Gut (9) befindet. Die Prozesskammer (8) kann ganz oder teilweise mittels Vakuumpumpe (10) evakuiert und mit dem sterilisierenden Gasgemisch (14) gefüllt werden, so dass das zu sterilisierende Gut (9) in einer vordefinierten Zeit dem sterilisierenden Gasgemisch (14) zur Wirkung ausgesetzt werden kann. Dieser Vorgang kann mehrfach wiederholt werden, um insbesondere Güter mit engen Kapillaren und Spalten sicher zu sterilisieren. Auch kann das zu sterilisierende Gut (9) in einer Sterilverpackung mit einer gasdurchlässigen Membran (Tyvek) dem sterilisierenden Gasgemisch (14) zur Wirkung ausgesetzt werden. Auch ist es möglich das sterilisierende Gasgemisch (14) durch die Prozesskammer (8) durchströmen zu lassen und dann zu Entsorgen bzw. das sterilisierende Gasgemisch (14) über eine Stell- und Regeleinheit (13) im Kreislauf wiederum durch die Plasmaquelle (2) zu führen, so dass eine höhere Konzentration an bioziden Spezies im sterilisierenden Gasgemisch (14) erzielt werden kann.

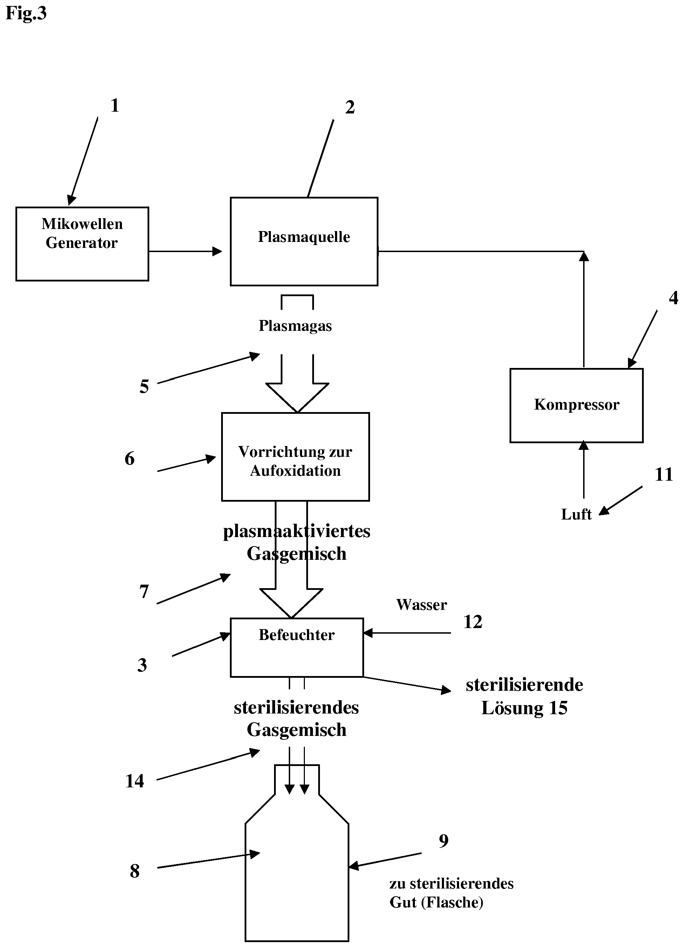

Fig. 3 zeigt eine Vorrichtung, die mittels einer Plasmaquelle (2) biozides, sterilisierendes Gasgemisch (14) und eine sterilisierende Lösung (15) erzeugt und eine Prozesskammer (8), die aus dem zu sterilisierenden Gut (9) selbst besteht.

Die Vorrichtung besteht aus einem Mikrowellengenerator (1), einer Plasmaquelle (2), einem Kompressor (4), einem Befeuchter (3), einer Vorrichtung zur Oxidation von NO (6) und einer Prozesskammer (8).

Die Prozesskammer (8) kann somit auch teilweise oder ganz aus dem zu sterilisierenden Gut (9) selbst gebildet werden, wie z.B. bei Behältnissen und Schläuchen. Solche Behältnisse und Schläuche können z.B. Kanister, Becher, Flaschen sowie Rohre und Medizinprodukte, wie Endoskope und Katheter, sein.

### Bezugszeichenliste

- 1: Mikrowellengenerator
- 2: Plasmaquelle
- 3: Befeuchter
- 4: Kompressor
- 5: heißes Plasma
- 6: Kühlvorrichtung
- 7: plasmaaktiviertes Gasgemisch
- 8: Prozesskammer
- 9: zu sterilisierendes Gut
- 10: Vakuumpumpe
- 11: Luft
- 12: Wasser
- 13: Steuer- und Regeleinrichtung
- 14: sterilisierendes Gasgemisch
- 15: sterilisierende Lösung

## Patentansprüche

1. Verfahren zur Dekontamination und Sterilisation von Gegenständen, **gekennzeichnet durch** folgende Schritte:
a) Erzeugen eines plasmaaktivierten Gasgemischs mit mindestens 0,3 % NO₂ - Anteil
b) Inkontaktbringen des in Schritt a) erzeugten plasmaaktivierten Gasgemischs mit Wasser in einem oder mehreren seiner Aggregatzustände,
c) Inkontaktbringen des unter Punkt b) erzeugten Gasgemischs mit den zu dekontaminierenden bzw. sterilisierenden Gegenständen,
**dadurch gekennzeichnet, dass**
die Erzeugung eines plasmaaktivierten Gasgemischs in Schritt a) die Schritte umfasst,
a1) Erzeugung eines heißen Luftplasma, wobei das Luftplasma eine Gastemperatur von mindestens 1200 °C aufweist, aus Luft als Prozessgas, welches reaktive Stickstoff- und Sauerstoffspezies bildet, wobei
a2) NO zu NO₂ bei Temperaturen unterhalb von 400 °C oxidiert wird.

2. Verfahren nach Anspruch 1, **dadurch gekennzeichnet, dass** sich durch das Inkontaktbringen des plasmaaktivierten Gasgemischs mit Wasser oder Wasserdampf bis zur Sättigung eine sterilisierende Lösung bildet, die mit den zu sterilisierenden Gegenständen in Kontakt gebracht wird.

3. Verfahren nach einem der Ansprüche 1 oder 2, **dadurch gekennzeichnet, dass**
a) die Reaktion des plasmaaktivierten Gasgemisches mit Wasser in einem Puffergefäß erfolgt oder dass das plasmaaktivierte Gasgemisch durch das Wasser durchgeleitet wird und/oder
b) das mit Wasser in Kontakt gebrachte Gasgemisch entweder über die zu sterilisierenden Gegenstände geleitet wird oder sich die Gegenstände in einer Prozesskammer befinden, in die das mit Wasser in Kontakt gebrachte Gasgemisch eingeleitet wird und/oder
c) dass im Gasgemisch gebildete Verbindungen durch Mikrokondensation auf der Oberfläche der Gegenstände abgeschieden werden.

4. Verfahren nach einem der Ansprüche 1 bis 3, **dadurch gekennzeichnet, dass** die Verweilzeit der zu sterilisierenden Gegenstände im sterilisierenden Gasgemisch zwischen 2 Sekunden und 60 Min. beträgt.

5. Verfahren nach einem der Ansprüche 1 bis 4, **dadurch gekennzeichnet, dass** die Erzeugung des Plasmas bei Atmosphärendruck erfolgt.

6. Verfahren nach einem der Anspräche 1 bis 5, **dadurch gekennzeichnet, dass** eine Prozesskammer vor Einleiten des sterilisierenden Gasgemisches evakuiert wird und die Befüllung der Prozesskammer bis zum Atmosphärendruck und höher erfolgt

7. Verfahren nach einem der Ansprüche 1 bis 6, **dadurch gekennzeichnet, dass** während der Wirkphase des sterilisierenden Gasgemisches eine mehrfache Druckänderung stattfindet.

8. Verfahren nach Anspruch 1 und 2, **dadurch gekennzeichnet, dass** Luft mit der sterilisierenden Lösung befeuchtet wird und das sich hieraus bildende Gasgemisch mit den zu dekontaminierenden Gegenständen in Kontakt gebracht wird.

9. Vorrichtung zur Durchführung des Verfahrens nach Anspruch 1 bis 8, umfassend eine Zuführvorrichtung für Luft (4), eine Plasmaquelle (2) zur Erzeugung eines Plasmas (5), eine Kühlvorrichtung zur Oxidation des Plasmagases (6), eine Einrichtung (3) zum Inkontaktbringen des plasmaaktivierten Gasgemischs (7) mit Wasser oder Wasserdampf (12) sowie eine Vorrichtung zur Aufnahme des zu sterilisierenden Gegenstands (9),
**dadurch gekennzeichnet, dass**
die Plasmaquelle (2) dazu ausgebildet ist ein Plasma (5) mit einer Gastemperatur von mindestens 1200 °C zu erzeugen und die Kühlvorrichtung derart ausgebildet ist das Plasmagas (6) auf unter 400 °C zur Oxidation des Plasmagases (6) abzukühlen.

10. Vorrichtung nach Anspruch 9, **dadurch gekennzeichnet, dass** die Plasmaquelle (2) eine mikrowellenangeregte Plasmaquelle ist.

11. Vorrichtung nach 9 oder 10, **dadurch gekennzeichnet, dass** die Vorrichtung eine Prozesskammer (8) zur Aufnahme der zu sterilisierenden Gegenstände umfasst.

12. Vorrichtung nach Anspruch 11, **dadurch gekennzeichnet, dass** die Prozesskammer (8) als Vakuum- und Druckkammer ausgeführt ist.

13. Vorrichtung nach einem der Ansprüche 9 bis 12, **dadurch gekennzeichnet, dass** sich am Ausgang der Plasmaquelle eine Düse befindet.

14. Vorrichtung nach einem der Ansprüche 9 bis 13, **dadurch gekennzeichnet, dass** die Vorrichtung ein Gerät zur Eindüsung von Wasser oder Wasserdampf als Einrichtung (3) zum Inkontaktbringen des plasmaaktivierten Gasgemischs (7) mit Wasser oder Wasserdampf umfasst.

15. Verwendung der Vorrichtung nach einem der Ansprüche 9 bis 14 zur Dekontamination und / oder Sterilisation von Gegenständen, insbesondere von
a) thermolabilen Gegenständen oder
b) Lebensmitteln oder
c) strukturierten Gegenständen mit engen Kapillaren oder Spalten oder
d) in gasdurchlässigen Verpackungen befindlichen Gegenständen oder
e) offenen Behältnissen, wie Flaschen und Preforms.

## Claims

1. A method for decontaminating and sterilizing objects, **characterized by** the following steps:
(a) generating a plasma-activated gas mixture containing at least 0,3 % NO₂
(b) contacting the plasma-activated gas mixture generated in step (a) with water in one or more of its states of aggregation,
(c) contacting the gas mixture generated according to point (b) with the objects to be decontaminated or sterilized
**characterized in that**
the generation of a plasma-activated gas mixture in step a) comprises the steps
a 1) generating a hot air plasma, wherein the air plasma has a gas temperature of at least 1200°C, from air as a process gas which forms reactive nitrogen and oxygen species, wherein
a2) NO is oxidized to NO₂ at temperatures below 400°C.

2. The method according to claim 1, **characterized in that** a sterilizing solution is formed by contacting the plasma-activated gas mixture with water or water vapor until saturation, which solution is contacted with the objects to be sterilized.

3. The method according to one of claims 1 or 2, **characterized in that**
(a) the reaction of the plasma-activated gas mixture with water takes place in a buffer vessel or that the plasma-activated gas mixture is passed through the water; and/or
(b) the gas mixture brought into contact with water is either passed over the objects to be sterilized or the objects are in a process chamber into which the gas mixture brought into contact with water is introduced; and/or
c) that compounds formed in the gas mixture are deposited on the surface of the objects by micro condensation.

4. The method according to one of claims 1 to 3, **characterized in that** the dwell time of the objects to be sterilized in the sterilizing gas mixture is between 2 seconds and 60 minutes.

5. The method according to one of claims 1 to 4, **characterized in that** the plasma is generated at atmospheric pressure.

6. The method according to one of claims 1 to 5, **characterized in that** a process chamber is evacuated before the sterilizing gas mixture is introduced and the filling of the process chamber is carried out up to atmospheric pressure and higher.

7. The method according to one of claims 1 to 6, **characterized in that** a multiple pressure change takes place during the active phase of the sterilizing gas mixture.

8. The method according to claim 1 and 2, **characterized in that** air is moistened with the sterilizing solution and the gas mixture formed therefrom is brought into contact with the objects to be decontaminated.

9. A device for carrying out the method according to claim 1 to 8, comprising a supply device for air (4), a plasma source (2) for generating a plasma (5), a cooling device for oxidizing the plasma gas (6), a device (3) for contacting the plasma-activated gas mixture (7) with water or water vapor (12) and a device for receiving the object (9) to be sterilized,
**characterized in that**
the plasma source (2) is configured to generate a plasma (5) with a gas temperature of at least 1200°C and the cooling device is configured to cool the plasma gas (6) to below 400°C for oxidation of the plasma gas (6).

10. The device according to claim 9, **characterized in that** the plasma source (2) is a microwave excited plasma source.

11. The device according to 9 or 10, **characterized in that** the device comprises a process chamber (8) for receiving the objects to be sterilized.

12. The device according to claim 11, **characterized in that** the process chamber (8) is configured as a vacuum and pressure chamber.

13. The device according to one of the claims 9 to 12, **characterized in that** a nozzle is located at the outlet of the plasma source.

14. The device according to one of the claims 9 to 13, **characterized in that** the device comprises a device for injecting water or water vapor as a device (3) for contacting the plasma-activated gas mixture (7) with water or water vapor.

15. A use of the device according to one of claims 9 to 14 for
decontaminating and / or sterilizing objects, in particular
(a) thermolabile objects; or
(b) food; or
(c) structured objects with narrow capillaries or gaps; or
(d) objects contained in gas-permeable packaging; or
(e) open containers, such as bottles and preforms.

## Revendications

1. Procédé de décontamination et de stérilisation d'objets, **caractérisé par** les étapes suivantes :
a) génération d'un mélange gazeux activé par un plasma avec une proportion d'au moins 0,3 % de NO₂,
b) mise en contact du mélange gazeux activé par un plasma, généré à l'étape a) avec de l'eau dans un ou plusieurs de ses états d'agrégation,
c) mise en contact du mélange gazeux généré au point b) avec les objets à décontaminer ou à stériliser,
**caractérisé en ce que**
la génération d'un mélange gazeux activé par un plasma à l'étape a) comprend les étapes suivantes,
a1) génération d'un plasma d'air chaud, dans lequel le plasma d'air présente une température de gaz d'au moins 1200 °C, à partir d'air servant de gaz de traitement, lequel forme des substances réactives à base d'azote et d'oxygène, dans lequel
a2) le NO est oxydé en NO₂ à des températures inférieures à 400 °C.

2. Procédé selon la revendication 1, **caractérisé en ce qu'**une solution stérilisante se forme du fait de la mise en contact du mélange gazeux activé par un plasma avec de l'eau ou de la vapeur d'eau jusqu'à la saturation, qui est mise en contact avec les objets à stériliser.

3. Procédé selon l'une des revendications 1 ou 2, **caractérisé en ce que**
a) la réaction du mélange gazeux activé par un plasma avec de l'eau s'effectue dans un récipient tampon ou que le mélange gazeux activé par un plasma passe à travers l'eau et/ou
b) le mélange gazeux mis en contact avec de l'eau est dirigé au-dessus des objets à stériliser ou bien les objets se trouvent dans une chambre de traitement, à l'intérieur de laquelle le mélange gazeux mis en contact avec de l'eau est introduit et/ou
c) que les composés formés dans le mélange gazeux sont déposés par microcondensation à la surface des objets.

4. Procédé selon l'une des revendications 1 à 3, **caractérisé en ce que** le temps de séjour des objets à stériliser dans le mélange gazeux stérilisant se situe entre 2 secondes et 60 min.

5. Procédé selon l'une des revendications 1 à 4, **caractérisé en ce que** la génération du plasma se fait à la pression atmosphérique.

6. Procédé selon l'une des revendications 1 bis 5, **caractérisé en ce qu'**une chambre de traitement est évacuée avant l'introduction du mélange gazeux stérilisant et que le remplissage de la chambre de traitement se fait jusqu'à la pression atmosphérique et au-delà.

7. Procédé selon l'une des revendications 1 bis 6, **caractérisé en ce qu'**une variation de pression a lieu plusieurs fois durant la phase où le mélange gazeux stérilisant agit.

8. Procédé selon la revendication 1 et 2, **caractérisé en ce que** l'air est humidifié par la solution stérilisante et le mélange gazeux en découlant est mis en contact avec les objets à décontaminer.

9. Dispositif pour mettre en œuvre le procédé selon la revendication 1 à 8, comprenant un dispositif d'alimentation en air (4), une source de plasma (2) servant à générer un plasma (5), un dispositif de refroidissement servant à oxyder le gaz plasmagène (6), une installation (3) servant à mettre en contact le mélange gazeux activé par un plasma (7) avec de l'eau ou de la vapeur d'eau (12), ainsi qu'un dispositif servant à recevoir l'objet à stériliser (9),
**caractérisé en ce que**
la source de plasma (2) est conçue de manière à générer un plasma (5) avec une température de gaz d'au moins 1200 °C et le dispositif de refroidissement est conçu de façon à refroidir le gaz plasmagène (6) à une température inférieure à 400 °C pour oxyder le gaz plasmagène (6).

10. Dispositif selon la revendication 9, **caractérisé en ce que** la source de plasma (2) est une source de plasma excitée par des micro-ondes.

11. Dispositif selon la revendication 9 ou 10, **caractérisé en ce que** le dispositif comprend une chambre de traitement (8) servant à recevoir les objets à stériliser.

12. Dispositif selon la revendication 11, **caractérisé en ce que** la chambre de traitement (8) est réalisée sous la forme d'une chambre à vide et de compression.

13. Dispositif selon l'une des revendications 9 à 12, **caractérisé en ce qu'**une buse se trouve à la sortie de la source de plasma.

14. Dispositif selon l'une des revendications 9 à 13, **caractérisé en ce que** le dispositif comprend un appareil d'injection d'eau ou de vapeur d'eau sous la forme d'une installation (3) servant à mettre en contact le mélange gazeux activé par un plasma (7) avec de l'eau ou de la vapeur d'eau.

15. Utilisation du dispositif selon l'une des revendications 9 à 14 pour décontaminer et/ou stériliser des objets, notamment
a) des objets thermolabiles ou
b) des denrées alimentaires ou
c) des objets structurés avec des fentes ou des capillaires étroits ou
d) des objets se trouvant dans des emballages perméables aux gaz ou
e) des récipients ouverts tels que des bouteilles et des préformes.
